# EUROPÄISCHE PATENTANMELDUNG

(11) **EP 1 260 281 A2**
(43) Veröffentlichungstag der Anmeldung: **27.11.2002**
(21) Anmeldenummer: 02009075.9
(22) Anmeldetag: 23.04.2002
(51) Int. Cl.: B08B 9/027, A61L 2/07, C02F 9/00

(54) **Verfahren zum Desinfizieren und Reinigen von Wasseraufbereitungsanlagen**

(30) Priorität: 01.05.2001 DE 10121206
(71) Anmelder: Kopper, Iris, 44227 Dortmund (DE)
(72) Erfinder: Pürschel Dieter, D-44227 Dortmund (DE)
(74) Vertreter: Grosse, Wolf-Dietrich, Dipl.-Ing.

(57) **Zusammenfassung**

Ein Verfahren zum Desinfizieren und Reinigen von Wasseraufbereitungsanlagen. Die Anlage weist eine Wasserzuführleitung, an die Sterilfilter (3), Durchlaufelemente, Druckerhöhungs- (5), Druckregel- (2), Lüftungs- (6,7), Carbonisier- (11) und Wasserentnahmeeinrichtungen (14) angeschlossen sind, auf. Die Durchgangsleitungen dieser Einrichtungen und deren Verbindungsleitungen werden, nach Trennung von der Wasserzufuhr mit einem Heissdampf durchströmt und beaufschlagt, der unter starkem Druck steht.

## Beschreibung

Die Erfindung betrifft ein Verfahren zum Desinfizieren und Reinigen von Wasseraufbereitungsanlagen mit einer Wasserzuführleitung, der Sterilfilter, Durchlaufkühlelemente, Druckerhöhungs-, Druckregel-, Lüftungs-, Carbonisier- und Wasserentnahmeeinrichtungen vor- bzw. nachgeordnet sind.

Bei Wasseraufbreitungsanlagen in öffentlichen Gebäuden, insbes. Krankenhäusern, Altenheimen, Sportanlagen u.dergl. dringen häufig schädliche Keime, Bakterien uns andere Verunreinigungen über den Zapfhahn in das Netz der Verbindungsleitungen in die Durchgangsleitungen der Aufbereitungseinrichtungen ein und setzen sich in diesen mit der Folge fest, dass das zugeführte Frischwasser, mit schädlichen Keimen und Bakterien durchsetzt aus dem Zapfhahn austritt und getrunken oder als Spülwasser verwandt ggfs zu gesundheitlichen Schäden für die Benutzer führt.

Der Erfindung liegt die Aufgabe zugrunde, ein Verfahren zum Desinfizieren und Reinigen für Wasseraufbereitungsanlagen der genannten Art zu schaffen, das das Auftreten solcher Keime bzw.Bakterien und Verunreinigungen im, in solchen Wasseraufbereitungsanlalagen bzw. in dem, aus diesen austretenden, aufbereiteten Wasser zuverlässig verhindert.

Diese Aufgabe wird dadurch gelöst, dass die, das aufbereitete Wasser führenden Durchgangsleitungen der Druckregel-, Lüftungs-, und Carbonisiereinrichtungen sowie deren Verbindungsleitungen, nach Trennung von der Wasserzufuhr mit, unter Druck stehendem Heissdampf durchströmt und beaufschlagt werden, dabei können, an die Wasserzufuhrleitung angeschlossene Filtereinrichtungen nach Trennung von dieser, im Gegenstrom von dem Heissdampf beaufschlagt werden.

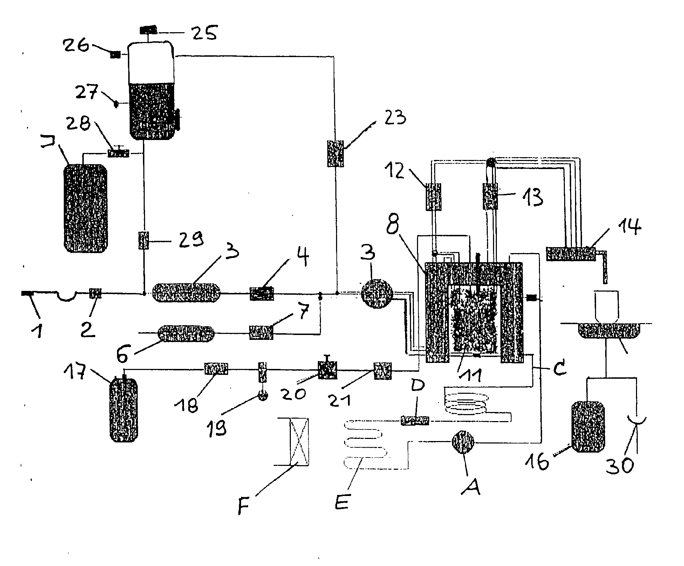

Die Erfindung wird anhand des, in der Zeichnung dargestellten Ausführungsbeispiels näher erläutert.

Wie aus der Zeichnung zu ersehen, ist in der Verbindungsleitung hinter dem Wasserleitungsanschluss 1 ein Wasserdruckminderer 2 und hinter diesem ein Sterilfilter 3 angeordnet. Die Verbindungsleitung führt dann über ein Magnetventil 4 über eine Druckerhöhungspumpe 5 in den Carbonator 11. Weiter führt dann zu diesem Carbonator 11 eine Verbindungsleitung von der Luftpumpe 6 über ein Magnetventil 7 und eine Druckerhöhungspumpe 5. Weiter führt eine Verbindungsleitung von einer C02-Flasche 17 über ein Magnetventil 18, einen Druckminderer 19 unter Zwischenschaltung eines Absperrhahns 20 und eines Rückschlagventils 21 zu dem Carbonator 11. Eine, aus Kälteanlagenkompressor A, Verflüssiger E, Trockner D und Lüfter F bestehende Kühleinrichtung ist über ein Kapillarrohr rohr C mit dem Carbonator 11 und der Getränkekühlschlange 8 kühlverbunden.

Das, aus dem Wasserzuleitungsanschluss 1 kommende Wasser wird in der Wasserkühlschlange gekühlt und gelangt dann über eine Verbindungsleitung, die Magnetventil 12 aufweist, in den Kompensatorhahn 14, dem es als stilles kühles Wasser entnommen werden kann, während das, in dem Carbonator 11 carbonisierte, gekühlte Wasser über ein Magnetventil 13 ebenfalls in den Kompensatorhahn 14 gelangt und diesem als Sprudelwasser entnommen werden kann. Unter dem Kompensatorhahn 14 befindet sich eine Tropfmulde 15, die mit einem Restwasserauffangbehälter 16 und einem Kanalanschluss 30 verbunden ist.

Der Dampfkessel H weist Ueberdruckventil 25, einen Druckwächter 26 und eine Druckanzeige 27 auf. Er ist über eine Verbindungsleitung mit der, von dem Wasserleitungsanschluss 1 kommenden Verbindungsleitung unter Zwichenschaltung eines Magnetventils 29 sowie über einen Abzweig von dieser Leitung und einen Abschlämmhahn 28 an einen Abschlämmbehälter J angeschlossen.

Von dem Dampfdom des Dampfkessels H führt über ein Magnetventil 23 eine Verbindungsleitung in die, vom Wasserleitungsanschluss 1 kommende Verbindungsleitung, die mit der Getränkekühlschlange 8 und dem Carbonator 11 verbunden ist.

Zum Reinigen und Desinfizieren wird bei gleichzeitigem Sperrabschluss der Zufuhrleitungen von Wasser und C02 mittels der Magnetventile 4 und 7 sowie des Rückschlagventils 21 und Oeffnung des Magnetventils des Kompensatorhahns 14 Heissdampf aus dem Dampfdom des Dampfkessels H durch das, hinter der Druckerhöhungspumpe 5 befindliche Leitungs- und Ventilsystem gedrückt und damit dessen Innenwandungen von Rückständen befreit und durch die Wirkung der Dampfhitze desinfiziert.

Bezugszeichenliste
- A: Kälteanlagenkompressor
- C: Kapillarrohr
- D: Trockner
- E: Verflüssiger
- F: Lüfter
- H: Dampfkessel
- J: Abschlämmbehälter
- 1: Wasserleitungsanschluss
- 2: Wasserdruckminderer
- 3: Sterilfilter
- 4: Magnetventil
- 5: Druckerhöhungspumpe
- 6: Luftpumpe
- 7: Magnetventil
- 8: Getränkekühlschlange
- 11: Carbonator
- 12: Magnetventil
- 13: Magnetventil
- 14: Kompensatorhahn
- 15: Tropfmulde
- 16: Restwasserauffangbehälter
- 17: CO2-Flasche
- 18: Magnetventil
- 19: Druckminderer
- 20: Absperrhahn
- 21: Rückschlagventil
- 23: Magnetventil
- 25: Ueberdruckventil
- 26: Druckwächter
- 27: Druckanzeige
- 28: Abschlämmhahn
- 29: Magnetventil
- 30: Kanalanschluss

## Patentansprüche

1. Verfahren zum Desinfizieren und Reinigen von Wasseraufbereitungsanlagen mit einer Wasserzuführleitung, der Sterilfilter, Durchlaufkühlelemente, Druckerhöhungs-, Druckregel-, Lüftungs-, Carbonisier- und Wasserentnahmeeinrichvor- bzw. nachgeordnet sind,
**dadurch gekennzeichnet,**
**dass** die, das aufbereitete Wasser führenden Durchgangsleitungen der Druckregel-, Lüftungs-, und Carbonisiereinrichtungen sowie deren Verbindungsleitungen, nach Trennung von der Wasserzufuhr mit, unter Druck stehendem Heissdampf durchströmt und beaufschlagt werden.

2. Verfahren nach Anspruch 1,
**dadurch gekennzeichnet,**
**dass**, an die Wasserzufuhrleitung angeschlossene Filtereinrichtungen nach Trennung von dieser, im Gegenstrom von dem Heissdampf beaufschlagt werden.
